# EUROPÄISCHE PATENTSCHRIFT

(11) **EP 1 389 193 B1**
(45) Veröffentlichungstag und Bekanntmachung des Hinweises auf die Patenterteilung: **19.10.2005**
(21) Anmeldenummer: 02750945.4
(22) Anmeldetag: 17.05.2002
(51) Int. Cl.: C07D 295/08, C07D 295/10

(54) **VERFAHREN ZUR HERSTELLUNG VON BIPERIDEN I**
METHOD FOR PRODUCING BIPERIDEN I
PROCEDE DE PRODUCTION DE BIPERIDENE I

(30) Priorität: 18.05.2001 DE 10124452; 18.05.2001 DE 10124450
(43) Veröffentlichungstag der Anmeldung: 18.02.2004
(73) Patentinhaber: LABORATORIO FARMACEUTICO S.I.T. SPECIALITA' IGIENICO TERAPEUTICHE S.r.l., 27035 Mede (Pavia) (IT)
(72) Erfinder: KASTNER, Gerhard, 67067 Ludwigshafen (DE); SCHEIB, Klaus, 67125 Dannstadt-Schauernheim (DE)
(74) Vertreter: De Gregori, Antonella
(86) Internationale Anmeldenummer: PCT/EP2002/005498
(87) Internationale Veröffentlichungsnummer: WO 2002/094801

(56) Entgegenhaltungen:
- DE-B- 1 005 067
- US-A- 2 789 110

## Beschreibung

Die vorliegende Erfindung betrifft ein Verfahren zur Herstellung von Biperiden.

Biperiden ist ein bekanntes zentrales Anticholinergikum und wird zur Behandlung der Parkinsonschen Krankheit eingesetzt (Ullmanns Enzyklopädie der technischen Chemie, 4. Aufl., Band 21, Verlag Chemie, 1982, S. 627). Es handelt sich um ein Racemat aus 1-(Bicyclo[2.2.1]hept-5-en-2-yl(exo,R))-1-phenyl-3-piperidino-propanol(1,S) und 1-(Bicyclo[2.2.1]hept-5-en-2-yl(exo,S))-1-phenyl-3-piperidinopropanol(1,R) (Ia) und stellt eines von vier möglichen Enantiomerenpaaren (Ia-d) des Aminoalkohols 1-(Bicyclo-[2.2.1]hept-5-en-Z-yl)-1-phenyl-3-piperidino-1-propanol (I) dar.

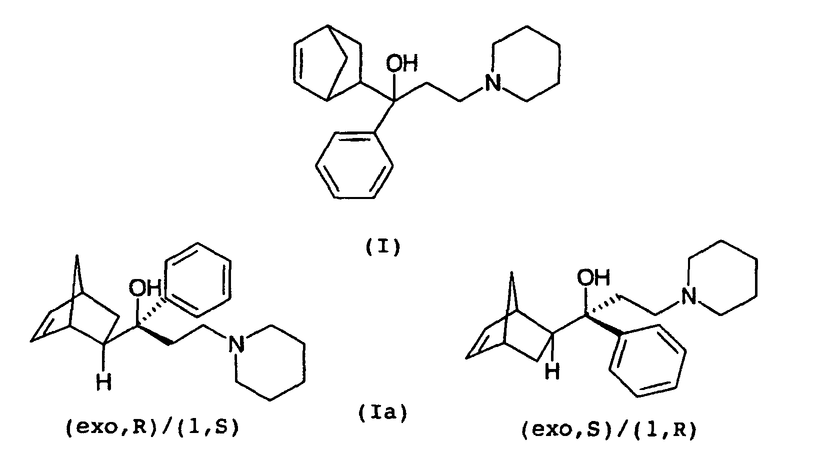

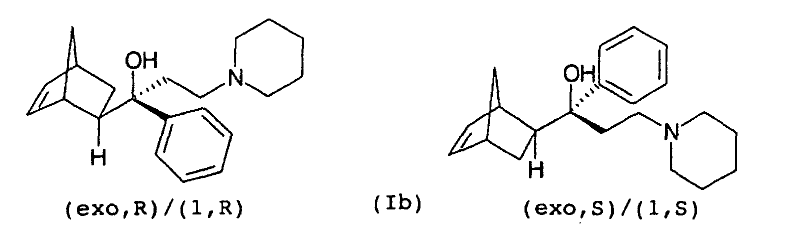

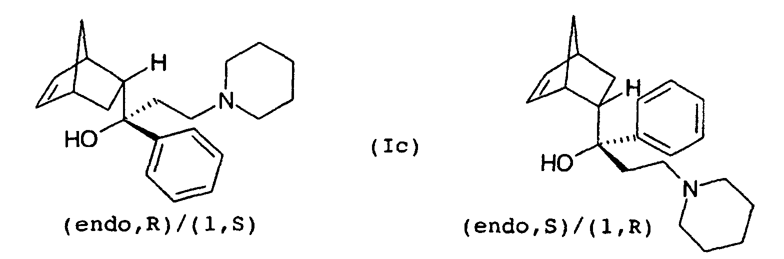

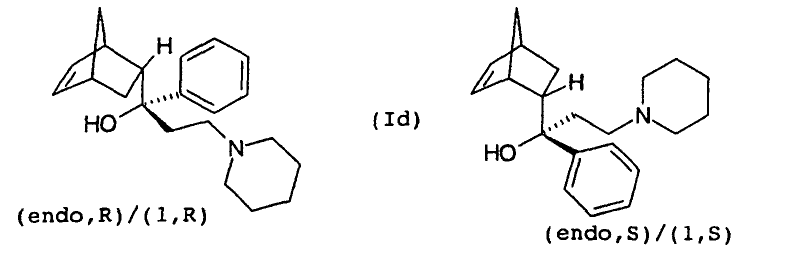

Die DE 1 005 067 und die US 2,789,110 beschreiben die Herstellung des Aminoalkohols I durch Umsetzung von 1-(Bicyclo[2.2.1]hept-5-en-2-yl)-3-piperidino-1-propanon (II) mit einem Phenylmagnesiumhalogenid. Die US 2,789,110 beschreibt zudem die Herstellung des Propanons II, ausgehend von 1-(Bicyclo[2.2.1]hept-5-en-2-yl)ethanon (III), Paraformaldehyd und Piperidinhydrochlorid in einer Reaktion nach Mannich sowie die Herstellung des Ethanons III aus Cyclopentadien und Methylvinylketon in einer Cycloaddition nach Diels und Alder.

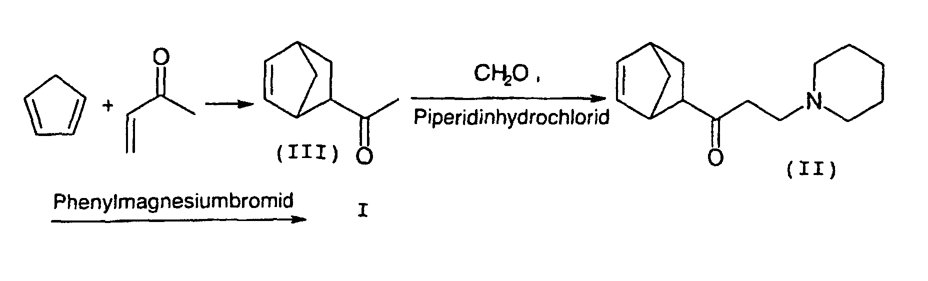

Weder die DE 1 005 067 noch die US 2,789,110 offenbaren, ob es sich bei dem so erhaltenen Aminoalkohol I um ein Isomerengemisch oder um ein reines Isomer handelt.

Das Edukt der Propanolherstellung, 1-(Bicyclo[2.2.1]hept-5-en-2-yl)-3-piperidino-1-propanon (II), kann in zwei isomeren Formen, als exo- oder als endo-Isomeres (II-exo, II-endo), vorliegen, wobei nur die exo-Form in der oben genannten Reaktion mit einem Phenylmagnesiumhalogenid Biperiden ergeben kann.

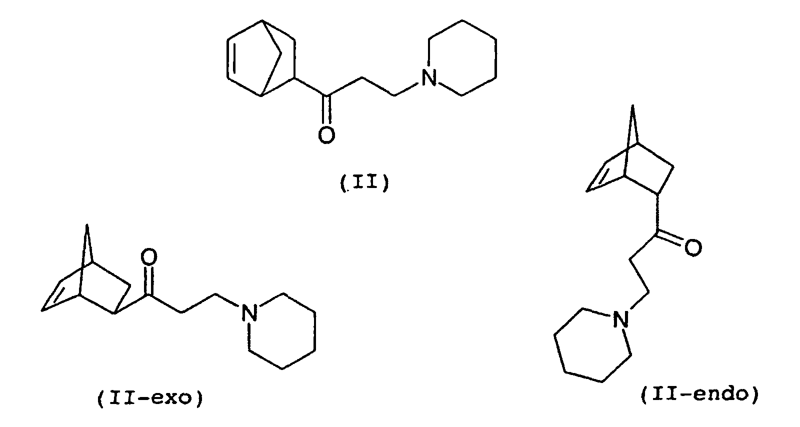

Die Strukturformeln des II-exo und des II-endo zeigen der Einfachheit halber jeweils nur eines von zwei möglichen Enantiomeren des exo- bzw. endo-Isomers. Im Folgenden bezieht sich die Bezeichnung II-exo bzw. II-endo jedoch auf das Enantiomerenpaar der exo- bzw. endo-Form.

Auch 1-(Bicyclo[2.2.1]hept-5-en-2-yl)ethanon (III), die Ausgangssubstanz für die Synthese des Propanons II, kann sowohl als exoals auch als endo-Isomeres vorliegen (III-exo, III-endo) und entsprechend führt nur die Umsetzung des exo-Isomeren in den Folgeschritten zu Biperiden.

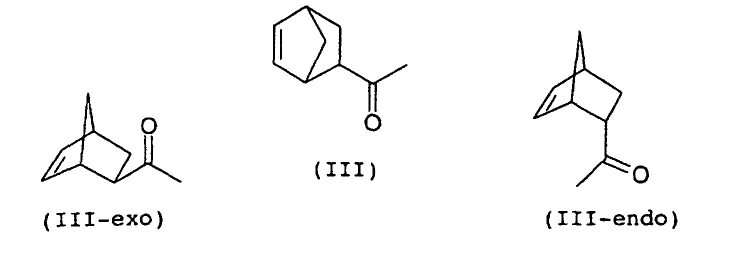

Die Strukturformeln des III-exo und des III-endo zeigen der Einfachheit halber jeweils nur eines von zwei möglichen Enantiomeren des exo- bzw. endo-Isomers. Im Folgenden bezieht sich die Bezeichnung III-exo bzw. III-endo jedoch auf das Enantiomerenpaar der exo- bzw. endo-Form.

Keiner der oben genannten Schriften lassen sich Angaben zur Konfiguration der eingesetzten Edukte III und Zwischenprodukte II entnehmen.

Es ist bekannt, dass das 1-(Bicyclo[2.2.1]hept-5-en-2-yl)ethanon (III) aus der Cycloaddition in einem exo/endo-Verhältnis von 1:4 erhalten wird (z.B. R. Breslow, U. Maitra, Tetrahedron Letters 1984, 25, 1239). Da der eingangs genannte Stand der Technik keinerlei Angaben zur Stereochemie des Ethanons III macht, ist davon auszugehen, dass das Ethanon III in diesem Isomerenverhältnis zur Herstellung des Aminoalkohols I eingesetzt wurde.

Die Herstellung von exo-1-(Bicyclo[2.2.1]hept-5-en-2-yl)ethanon (III-exo) ist 1965 von J.G. Dinwiddie und S.P. McManus beschrieben worden (J. Org. Chem., 1965, 30, 766). Dabei werden exo/endo-Gemische von 1-(Bicyclo[2.2.1]hept-5-en-2-yl)ethanon (III), in denen der endo-Anteil überwiegt, in Methanol in Gegenwart von Natriummethanolat erhitzt und isomerisieren zu Gemischen mit einem exo-Anteil von ca. 70 %. Aus diesen kann durch fraktionierte Destillation und gegebenenfalls Redestillation des Destillats exo-1-(Bicyclo[2.2.1]hept-5-en-2-yl)ethanon (III-exo) mit einem Reinheitsgrad von bis zu 95 % erhalten werden.

Versuche der Anmelderin haben gezeigt, dass auch bei der Verwendung von nahezu einheitlichem exo-1-(Bicyclo[2.2.1]hept-5-en-2-yl)ethanon (III-exo), d. h. von einem Ethanon III mit einem exo-Anteil von wenigstens 95 %, als Ausgangsmaterial sauberes Biperiden (Ia) in nur geringen Ausbeuten erhalten werden kann, wobei vor allem die Umsetzung des 1-(Bicyclo[2.2.1]hept-5-en-2-yl)-3-piperidino-1-propanons (II) mit einem Phenylmagnesiumhalogenid mit einer sehr schlechten Ausbeute an Biperiden (Ia) verläuft. Unter sauberem Biperiden versteht man ein Biperiden (Ia) mit einer Reinheit von wenigstens 99,0 %, wie sie für pharmazeutische Anwendungen in der Regel erforderlich ist.

Der vorliegenden Erfindung liegt die Aufgabe zugrunde, ein Verfahren zur Herstellung von Biperiden bereit zu stellen, das dieses in einer höheren Ausbeute liefert. Unter Biperiden soll eine Substanz der Strukturformel Ia verstanden werden.

Die Aufgabe konnte durch ein Verfahren zur Herstellung von Biperiden (Ia) gelöst werden, bei dem man ein exo/endo-Gemisch von 1-(Bicyclo[2.2.1]hept-5-en-2-yl)-3-piperidino-1-propanon (II) mit einem Isomerenverhältnis der exo-Form zur endo-Form von wenigstens 2,5:1, vorzugsweise von wenigstens 3,0:1 und insbesondere von etwa 3,5-4,0:1 mit Diphenylmagnesium umsetzt, wobei man ein Biperiden-haltiges Isomerengemisch erhält und aus diesem Biperiden (Ia) gewinnt.

Bei den im erfindungsgemäßen Verfahren eingesetzten exo- und endo-Isomeren handelt es sich, wie bereits für das exo- und endo-Ethanon III-exo und III-endo sowie für das exo- und endo-Propanon II-exo und II-endo beschrieben, um Enantiomerenpaare. Um zu Biperiden (Ia) zu gelangen, das selbst ein Racemat ist, werden racemische Enantiomerengemische der Ausgangsstoffe und der Zwischenprodukte eingesetzt. Das erfindungsgemäße Verfahren kann jedoch auch auf reine Enantiomere sowie auf nicht racemische Enantiomerengemische angewendet werden.

Die Umsetzung von Diphenylmagnesium mit dem exo/endo-Gemisch des Propanons II erfolgt geeigneterweise in einem für Grignard-Reaktionen gängigen Lösungsmittel. Hierzu zählen Benzol, Toluol, Xylole, acyclische oder cyclische Ether mit 4 bis 6 C-Atomen, Gemische davon oder deren Mischungen mit aliphatischen oder alicyclischen Kohlenwasserstoffen wie n-Hexan bzw. Cyclohexan. Geeignete acyclische Ether sind z. B. Diethylether und tert-Butylmethylether, geeignete cyclische Ether sind z. B. Tetrahydrofuran und Dioxan. Bevorzugt verwendet man Diethylether, Tetrahydrofuran oder Dioxan oder deren Mischungen. Die Lösungsmittel werden in der Regel, wie für Grignard-Reaktionen üblich, wasserfrei eingesetzt.

In der Regel setzt man Diphenylmagnesium und 1-(Bicyclo[2.2.1]-hept-5-en-2-yl)-3-piperidino-1-propanon (II) in einem Molverhältnis im Bereich von 0,8:1 bis 3:1 ein, vorzugsweise im Bereich von 0,8:1 bis 2:1, insbesondere im Bereich von 0,8:1 bis 1,5:1. Besonders bevorzugt wird II mit Diphenylmagnesium in nahezu äquimolaren Mengen umgesetzt.

In der Regel legt man Diphenylmagnesium in Form einer Lösung in einem der oben genannten organischen Lösungsmittel vor und fügt das Propanon II bei einer Temperatur im Bereich von -20 °C bis zur Siedetemperatur, vorzugsweise im Bereich von -10 °C bis 90 °C und besonders bevorzugt im Bereich von 0 °C bis 70 °C hinzu. Dabei wird Diphenylmagnesium in der Regel in einer Konzentration im Bereich von 0,1 bis 10 mol/l, vorzugsweise im Bereich von 0,1 bis 3 mol/l und besonders bevorzugt im Bereich von 0,2 bis 2 mol/l eingesetzt.

Die Zugabe des Propanons II kann in einer Portion oder vorzugsweise über einen Zeitraum von wenigen Minuten bis zu mehreren Stunden, z. B. 5 Minuten bis 5 Stunden, erfolgen. Die Zugabe des Propanons II erfolgt entweder in Form einer Lösung in einem der oben genannten, für Grignard-Reaktionen geeigneten, inerten Lösungsmittel oder vorzugsweise in Reinform. Bei der Zugabe als Lösung beträgt die Konzentration des Propanons II in der Regel 0,1 bis 20 mol/l, vorzugsweise 1 bis 15 mol/l. Zur Vervollständigung der Reaktion wird das Reaktionsgemisch üblicherweise 15 Minuten bis 5 Stunden, speziell 30 Minuten bis 2 Stunden bei einer Temperatur im Bereich von -20 °C bis zur Siedetemperatur des Reaktionsgemischs, vorzugsweise im Bereich von -10 °C bis 90 °C und besonders bevorzugt im Bereich von 10 °C bis 80 °C belassen, wobei man vorzugsweise zur besseren Durchmischung rührt. Die Aufarbeitung erfolgt wie für Grignard-Reaktionen üblich wässrig-extraktiv, z. B. indem man das Reaktionsgemisch mit Wasser, einer wässrigen Ammoniumchloridlösung oder einer sauren wässrigen Lösung quencht, wobei man im letzten Fall den pH-Wert des entstandenen Gemischs anschließend alkalisch einstellt, das gequenchte Gemisch gegebenenfalls nach Abtrennung einer organischen Phase mit einem mit Wasser nicht mischbaren, zum Lösen des Produkts geeigneten Lösungsmittel extrahiert und aus dem Extrakt bzw. aus dem mit der organischen Phase vereinigten Extrakt das Lösungsmittel entfernt. Geeignete Lösungsmittel sind beispielsweise Aromaten wie Benzol oder Toluol, die oben genannten acyclischen Ether, Ester wie Ethylacetat oder chlorhaltige Aliphaten wie Dichlor- oder Trichlormethan.

Die Herstellung des in dem erfindungsgemäßen Verfahren eingesetzten Diphenylmagnesiums erfolgt in an sich bekannter Weise. Beispielsweise kann man ein Phenylmagnesiumhalogenid, z. B. Phenylmagnesiumchlorid, in einem geeigneten Lösungsmittel mit Dioxan versetzen, wobei unter der Verschiebung des Schlenk-Gleichgewichts Diphenylmagnesium und der entsprechende Magnesiumhalogenid-Dioxan-Komplex entstehen. Letzterer fällt in der Regel aus, wird aber bevorzugt nicht aus der Lösung entfernt. Geeignete Lösungsmittel sind im Allgemeinen acyclische und cyclische Ether mit vorzugsweise 4 bis 6 C-Atomen oder deren Mischungen mit aliphatischen, alicyclischen oder aromatischen Kohlenwasserstoffen. Geeignete acyclische Ether sind z. B. Diethylether und tert-Butylmethylether, ein geeigneter cyclischer Ether ist Tetrahydrofuran. Zu den geeigneten aliphatischen bzw. alicyclischen Kohlenwasserstoffen zählen insbesondere n-Hexan bzw. Cyclohexan, geeignete aromatische Kohlenwasserstoffe sind z. B. Benzol, Toluol und Xylole.

Dioxan wird in der Regel mindestens äquimolar im Verhältnis zum Phenylmagnesiumhalogenid eingesetzt; vorzugsweise setzt man Dioxan im Überschuss, beispielsweise in einem Überschuss von 50 bis 500 Mol-%, insbesondere von 100 bis 300 Mol-% und speziell von 100 bis 200 Mol-%, ein.

Die Zugabe des Dioxans zu der Lösung des Phenylmagnesiumhalogenids erfolgt in der Regel bei einer Temperatur im Bereich von -20 bis 60 °C, vorzugsweise im Bereich von -10 bis 40 °C.

Üblicherweise belässt man die nach Zugabe des Dioxans erhaltene Mischung noch 15 Minuten bis 2 Stunden, vorzugsweise 20 Minuten bis eine Stunde, in dem für die Zugabe des Dioxans genannten Temperaturbereich, bevor man sie ins erfindungsgemäße Verfahren einsetzt.

Sowohl die Herstellung von Diphenylmagnesium als auch die Umsetzung mit dem Propanon II nach Grignard erfolgen geeigneterweise unter einer Inertgasatmosphäre. Als Inertgase kommen Stickstoff und die Edelgase wie Argon sowie Gemische dieser Gase in Betracht.

Das aus der erfindungsgemäßen Umsetzung des Propanons II mit Diphenylmagnesium erhaltene Rohprodukt besteht im Wesentlichen aus den vier diastereomeren Enantiomerenpaaren Ia bis Id des 1-(Bicyclo[2.2.1]hept-5-en-2-yl)-1-phenyl-3-piperidino-1-propanols (I), wobei das Isomerenpaar Ia wenigstens 50 % der Isomerenmischung ausmacht. Bei Einsatz des Propanons II mit einem exo/endo-Verhältnis von etwa 3,5:1 enthält das Rohprodukt die Enantiomerenpaare in einem gaschromatographisch bestimmten Verhältnis von Biperiden (Ia) zu Form Ib zu Form Ic zu Form Id von etwa 10,4:3,4:3,0:1. Der Anteil von Biperiden (Ia) am Isomerengemisch beträgt hier 58 %.

Zur Isolierung des Biperidens (Ia) aus dem Diastereomerengemisch wird dieses unter Erwärmen, vorzugsweise bei einer Temperatur von 40 bis 80 °C, insbesondere von 50 bis 70 °C, in wässrigem Isopropanol, vorzugsweise in 70 bis 95%igem Isopropanol, besonders bevorzugt in 90%igem Isopropanol, gelöst und durch Zugabe von HCl in dem erwähnten Temperaturbereich, beispielsweise in Form einer Chlorwasserstofflösung in Isopropanol oder als Salzsäure, das Hydrochlorid ausgefällt und von der Lösung abgetrennt. Zur Vervollständigung der Salzbildung wird das Gemisch nach vollendeter Zugabe von HCl vorzugsweise bei einer Temperatur von 50 °C bis zur Siedetemperatur des Reaktionsgemisches etwa 0,5 bis 3 Stunden gerührt. Das Gemisch wird anschließend auf eine Temperatur im Bereich von 0 bis 30 °C abgekühlt, gegebenenfalls noch bis zu mehreren Stunden in diesem Temperaturbereich gerührt und das gebildete Hydrochlorid dann aus der Lösung isoliert. Die hier und im Folgenden gemachten %-Angaben bezüglich des Isopropanolgehalts beziehen sich auf das Volumen des Isopropanols bezogen auf das Gesamtvolumen des wasserhaltigen Lösungsmittels. HCl wird wenigstens äquimolar bezogen auf den Aminoalkohol I, vorzugsweise in einem Überschuss von 5 bis 50 Mol-% und besonders bevorzugt von 5 bis 20 Mol-%, eingesetzt. Zur weiteren Reinigung wird das Präzipitat mit wässrigem Isopropanol, vorzugsweise mit 70 bis 95%igem Isopropanol, besonders bevorzugt mit 90%igem Isopropanol, bei erhöhter Temperatur, vorzugsweise bei 40 bis 80 °C, insbesondere bei der Siedetemperatur des Gemischs, etwa 0,5 bis 3 Stunden gerührt und dann auf eine Temperatur im Bereich von 0 bis 30 °C abgekühlt, gegebenenfalls noch bis zu mehreren Stunden in diesem Temperaturbereich gerührt und dann das gereinigte Hydrochlorid abfiltriert. Zur weiteren Reinigung wird das so aufgereinigte Hydrochlorid bei erhöhter Temperatur, vorzugsweise bei 40 bis 60 °C, z. B. bei 50 °C, in einem C₁-C₂-Alkohol oder einem Gemisch davon, vorzugsweise in Methanol, mit einer wenigstens äquimolaren Menge einer geeigneten Base in die korrespondierende freie Base überführt. Geeignete Basen sind Alkali- oder Erdalkalihydroxide sowie Alkalicarbonate; bevorzugt werden Natrium- oder Kaliumhydroxid bzw. ihre wässrigen Lösungen und insbesondere Natriumhydroxid bzw. Natronlauge verwendet. Möglich ist aber auch die Verwendung wasserlöslicher organischer Basen, beispielsweise aliphatisch substituierter Amine mit 2 bis 8 C-Atomen. Zur Vervollständigung der Reaktion wird vorzugsweise nach vollendeter Zugabe der Base noch etwa 0,5 bis 3 Stunden in dem für die Zugabe der Base genannten Temperaturbereich gerührt. Das Reaktionsgemisch wird anschließend auf eine Temperatur im Bereich von 0 bis 30 °C abgekühlt und gegebenenfalls noch bis zu mehreren Stunden in diesem Temperaturbereich gerührt. Die Festsubstanz wird dann abfiltriert, mit Wasser gewaschen und anschließend bei erhöhter Temperatur, vorzugsweise bei 40 bis 80 °C, insbesondere bei der Siedetemperatur des Gemischs, in einem C₁-C₂-Alkohol oder einem Gemisch davon, vorzugsweise in Methanol, etwa 0,5 bis 3 Stunden gerührt, das Gemisch dann auf eine Temperatur im Bereich von 0 bis 30 °C abgekühlt, gegebenenfalls noch bis zu mehreren Stunden in diesem Temperaturbereich gerührt und dann der Feststoff in geeigneter Weise, z. B. über Filtration, abgetrennt. Auf diese Weise erhält man Biperiden (Ia) mit einer Reinheit von wenigstens 99,0 %.

Durch die erfindungsgemäße Verwendung von Diphenylmagnesium anstelle der im Stand der Technik eingesetzten Phenylmagnesiumhalogenide kann die Ausbeute an Biperiden (Ia) erheblich gesteigert werden.

Biperiden (Ia) kann anschließend mit einer pharmakologisch verträglichen Säure in üblicher Weise in sein Säureadditionssalz überführt werden. Geeignete Säuren sind beispielsweise Halogenwasserstoffsäuren, insbesondere Chlorwasserstoff bzw. Salzsäure, sowie organische Mono- oder Dicarbonsäuren wie Essigsäure, Oxalsäure, Maleinsäure, Fumarsäure, Milchsäure, Weinsäure, Adipinsäure oder Benzoesäure, weiterhin Phosphorsäure und Schwefelsäure sowie die in "Fortschritte der Arzneimittelforschung, Band 10, S. 224ff, Birkhäuser Verlag, Basel, Stuttgart, 1966" genannten Säuren. Üblicherweise kommt Biperiden (Ia) als Hydrochlorid in den Handel.

Das in die Grignard-Reaktion eingesetzte 1-(Bicyclo[2.2.1]hept-5-en-2-yl)-3-piperidino-1-propanon (II) erhält man durch Umsetzung von exo-1-(Bicyclo[2.2.1]hept-5-en-2-yl)ethanon (III-exo) in einer Reaktion nach Mannich in Anwesenheit einer Säure mit Piperidin und einer Formaldehydquelle oder mit dem Additionsprodukt von Piperidin und Formaldehyd, vorzugsweise in einem geeigneten Lösungsmittel.

Unter exo-1-(Bicyclo[2.2.1]hept-5-en-2-yl)ethanon (III-exo) soll im Folgenden ein Ethanon III mit einem exo-Anteil von wenigstens 96 % verstanden werden.

Geeignete Lösungsmittel sind insbesondere C₁-C₄-Alkanole, z. B. Methanol, Ethanol, n-Propanol, Isopropanol, sek-Butanol und Isobutanol. Vorzugsweise wird Isopropanol verwendet. Das exo-Ethanon (III-exo) und Piperidin werden in der Regel in einem Molverhältnis im Bereich von 0,5:1 bis 1,5:1, vorzugsweise von 1:1 eingesetzt. Formaldehyd liegt in der Regel im Überschuss vor, wobei der Überschuss bis zu 100 Mol-%, bezogen auf Piperidin, insbesondere bis zu 50 Mol-% betragen kann. Formaldehyd kann dabei sowohl gasförmig, als Formalin, als Trioxan als auch als Paraformaldehyd eingesetzt werden. Bevorzugt verwendet man Paraformaldehyd, insbesondere in Kombination mit Piperidiniumhydrochlorid. In einer bevorzugten Vorgehensweise werden das exo-Ethanon (III-exo), Piperidinhydrochlorid und Paraformaldehyd in Molverhältnissen von 1:0,9-1,2:1-1,4 miteinander umgesetzt. Als Lösungsmittel verwendet mann dann insbesondere C₁-C₄-Alkanole und speziell Isopropanol. Die Reaktionstemperatur liegt in der Regel im Bereich von 10°C bis zur Siedetemperatur des Gemischs. Vorzugsweise erhitzt man zum Rückfluss.

Die Aufarbeitung erfolgt in an sich bekannter Weise. Hierzu wird man gewöhnlich zuerst das Lösungsmittel bei vermindertem Druck entfernen und den Rückstand in Wasser aufnehmen. Die erhaltene wässrige Lösung wird man dann mit einem geeigneten organischen Lösungsmittel, d. h. mit einem mit Wasser nicht mischbaren, mäßig polaren Lösungsmittel, beispielsweise einem aliphatischen Ether mit 4 bis 6 C-Atomen, wie Diethylether, tert-Butylmethylether oder insbesondere Diisopropylether, extrahieren. Die Extraktion erfolgt in der Regel bei pH ≤ 7 und dient der Entfernung von Nebenprodukten. Insbesondere wird man zunächst eine Extraktion der nach Entfernung des Lösungsmittels und Verdünnen mit Wasser erhaltenen sauren wässrigen Mischung vornehmen, anschließend mit etwas Base abstumpfen und dann erneut extrahieren, wobei pH-Wert ≤ 7 eingehalten wird.

Dann wird man vorzugsweise die wässrige Phase durch Zugabe von Base in ein oder mehreren Stufen alkalisch stellen, vorzugsweise auf pH ≥ 7,5 insbesondere pH 7,5 bis 9 und speziell pH 8 bis 8,5, um das 1-(Bicyclo[2.2.1]-hept-5-en-2-yl)-3-piperidino-1-propanon (II), das noch als Säureadditionssalz vorliegt, in das freie Amin zu überführen. Als Basen kommen hierfür die üblichen anorganischen Basen in Betracht wie KOH, NaoH, Na₂CO₃, K₂CO₃ und dergleichen. Die wässrige Phase wird dann mit einem der oben genannten, mit Wasser nicht mischbaren, mäßig polaren Lösungsmittel, vorzugsweise Diisopropylether, ein oder mehrfach extrahiert. Zur Gewinnung des Propanons II aus dem Extrakt wird das Lösungsmittel, gegebenenfalls unter vermindertem Druck, entfernt. Zur weiteren Reinigung kann der Rückstand über eine Vakuumdestillation bei einem Druck von vorzugsweise weniger als 10 mbar, besonders bevorzugt von weniger als 5 mbar und insbesondere von weniger als 1 mbar, gereinigt werden. Das erhaltene Gemisch besteht aus exo- und endo-1-(Bicyclo[2.2.1]hept-5-en-2-yl)-3-piperidino-1-propanon (II) in einem Verhältnis von wenigstens 2,5:1, vorzugsweise von wenigstens 3,0:1 und insbesondere von 3,5-4,0:1.

Das zur Herstellung des 1-(Bicyclo[2.2.1]hept-5-en-2-yl)-3-piperidino-1-propanons (II) verwendete exo-1-(Bicyclo[2.2.1]hept-5-en-2-yl)ethanon (III-exo) erhält man durch die Umsetzung von Cyclopentadien und Methylvinylketon in einer Cycloaddition nach Diels und Alder. Cyclopentadien und Methylvinylketon werden in der Regel in einem Molverhältnis im Bereich von 3,0:1 bis 0,5:1 eingesetzt. Vorzugsweise werden sie äquimolar oder mit Cyclopentadien im Überschuss umgesetzt, wobei der Überschuss vorzugsweise 50 bis 150 Mol-% beträgt.

Die Reaktion wird in der Regel bei einer Temperatur im Bereich von 0 bis 60 °C, vorzugsweise im Bereich von 20 bis 40 °C durchgeführt.

Die Cycloaddition kann in einem für solche Reaktionen gängigen Lösungsmittel wie Diethylether, Benzol, Toluol oder Xylol oder auch ohne Lösungsmittel durchgeführt werden. Vorzugsweise wird kein Lösungsmittel verwendet.

Niedrig siedende Bestandteile, meist unumgesetzte Edukte und gegebenenfalls Lösungsmittel, werden in der Regel im Anschluss an die Cycloaddition bei erniedrigtem Druck, vorzugsweise bei 1 bis 150 mbar, destillativ entfernt. Das zurückbleibende Gemisch, das zu etwa 20 % aus exo- und zu etwa 80 % aus endo-1-(Bicyclo[2.2.1]hept-5-en-2-yl)ethanon besteht, wird vorzugsweise dann mit einem Alkali-C₁-C₄-alkoholat umgesetzt. Die Menge an Alkalialkoholat beträgt in der Regel 0,1 bis 5 Gew.-%, vorzugsweise 0,2 bis 2 Gew.-%, bezogen auf das Gesamtgewicht der Mischung. Vorzugsweise wird Natriummethanolat verwendet. Die zur Isomerisierung des Ethanons III erforderliche Temperatur liegt in der Regel im Bereich von 50 bis 110 °C, vorzugsweise im Bereich von 60 bis 100 °C. Hierzu erhitzt man häufig die Mischung unter vermindertem Druck zum Rückfluss, vorzugsweise bei einem Druck von 1 bis 100 mbar und insbesondere bei einem Druck von 5 bis 50 mbar.

Man wendet diese Bedingungen in der Regel 10 Minuten bis 5 Stunden, insbesondere 20 Minuten bis 3 Stunden und speziell 0,5 Stunden bis 2 Stunden an und beginnt dann das erhaltene Gemisch fraktioniert zu destillieren, wobei bevorzugt das exo-Isomer von III abdestilliert. Man geht davon aus, dass durch das Entfernen des exo-Isomeren aus dem Gleichgewicht die Isomerisierung des endo-Ethanons zur exo-Form gefördert wird. Die fraktionierte Destillation erfolgt in der Regel über eine Kolonne unter vermindertem Druck, vorzugsweise im Bereich von 1 bis 100 mbar, insbesondere von 1 bis 50 und speziell von 1 bis 20 mbar. Die Destillationstemperatur (Kopftemperatur) wird vorzugsweise auf 50 bis 100 °C und speziell auf 50 bis 80 °C eingestellt. Auf diese Weise erhält man exo-1-(Bicyclo[2.2.1]hept-5-en-2-yl)ethanon (III-exo) in einem Reinheitsgrad, der wenigstens 96 % beträgt. Durch Redestillation des Destillats erhält man das exo-Ethanon III-exo mit einem Reinheitsgrad von bis zu 100 %.

Die folgenden Beispiele dienen der Veranschaulichung der Erfindung, sind jedoch nicht einschränkend zu verstehen.

### Beispiele

### 1. Herstellung des Ausgangsmaterials

### 1.1 exo-1-(Bicyclo[2.2.1]hept-5-en-2-yl)ethanon (III-exo)

Zu 210,3 g Methylvinylketon wurden zügig 198,3 g Cyclopentadien gegeben. Nach beendeter Zugabe wurde die Reaktionslösung noch eine Stunde bei Raumtemperatur nachgerührt und dann bei einer Temperatur von 58 ° C und einem Druck von 20 mbar nicht umgesetztes Edukt destillativ entfernt. Der Eindampfrückstand, hauptsächlich bestehend aus einem Gemisch aus der exo- und der endo-Form von 1-(Bicyclo[2.2.1]hept-5-en-2-yl)ethanon (III) im Verhältnis von 1:4, wurde mit 5 g Natriummethanolat versetzt und eine Stunde bei einem Druck von 10 bis 20 mbar zum Rückfluss erhitzt. Das Reaktionsgemisch wurde anschließend über eine Kolonne bei einer Temperatur von 75 °C und einem Druck von 20 mbar destilliert. Erhalten wurden dabei 298,3 g (73 % der Theorie) exo-1-(Bicyclo[2.2.1]-hept-5-en-2-yl)ethanon (III-exo) in Form eines leicht gelblichen Öls.

### 1.2 1-(Bicyclo[2.2.1]hept-5-en-2-yl)-3-piperidino-1-propanon (II)

68,1 g exo-1-(Bicyclo[2.2.1]hept-5-en-2-yl)ethanon (III-exo), 60,8 g Piperidinhydrochlorid und 18 g Paraformaldehyd wurden in 140 ml Isopropanol fünf Stunden zum Rückfluss erhitzt. Das Lösungsmittel wurde im Vakuum entfernt und der Rückstand in 100 ml Wasser aufgenommen. Die Lösung wurde drei Mal mit jeweils 50 ml Diisopropylether gewaschen und dann mit 50%iger Natronlauge auf pH 10 eingestellt. Es wurde drei Mal mit jeweils 50 ml Diisopropylether extrahiert, die drei Extrakte vereinigt und das Lösungsmittel am Rotationsverdampfer entfernt. Der Eindampfrückstand wurde im Kugelrohr bei 75 °C im Hochvakuum bei 0,001 mbar destilliert. Als Destillat wurden 50,2 g (43 % der Theorie) eines Gemischs aus exo- und endo-1-(Bicyclo[2.2.1]hept-5-en-2-yl)-3-piperidino-1-propanon (II) im Verhältnis von 3,5:1 in Form eines farblosen Öls erhalten.

### 2. Beispiel A: Herstellung von Biperiden (Ia)

Zu 1500 g einer auf 0 °C abgekühlten, 25%igen Lösung von Phenylmagnesiumchlorid (375 g, 2,74 mol) in Tetrahydrofuran wurden unter Eisbadkühlung 603,6 g (6,85 mol) Dioxan gegeben, wobei sich ein weißer Niederschlag bildete. Es wurde noch 30 Minuten unter Eisbadkühlung gerührt und anschließend unter Eisbadkühlung 320 g (1,37 mol) des nach 1.2 erhaltenen 3,5:1-Gemischs aus den exo- und endo-Formen von 1-(Bicyclo[2.2.1]hept-5-en-2-yl)-3-piperidino-1-propanon (II) hinzugefügt. Nach beendeter Zugabe wurde das Eisbad entfernt und noch eine Stunde bei Raumtemperatur gerührt. Die Lösung wurde anschließend langsam zu 1500 ml eiskaltem Wasser gegeben und dann drei Mal mit jeweils 500 ml Toluol extrahiert. Die organischen Phasen wurden vereinigt, über Natriumsulfat getrocknet und am Rotationsverdampfer eingedampft. Der Eindampfrückstand, 433,8 g eines Gemischs, das im Wesentlichen aus den Formen Ia bis Id von 1-(Bicyclo[2.2.1]hept-5-en-2-yl)-1-phenyl-3-piperidino-1-propanol (I) im Verhältnis (GC) von 10,4:3,4:3,0:1 bestand, wurde in 3500 ml 90%igem Isopropanol heiß gelöst und die Lösung bei 60 °C mit 228 ml einer 6-molaren Lösung von Chlorwasserstoff in Isopropanol versetzt. Nach der Säurezugabe wurde eine Stunde bei 60 °C und dann noch 0,5 Stunden bei Rückflusstemperatur nachgerührt. Nach dem Abkühlen auf Raumtemperatur wurden die ausgefallenen Kristalle abgetrennt, mit 700 ml 90%igem Isopropanol gewaschen und im Vakuum bei 70 °C getrocknet. Das so erhaltene Hydrochlorid wurde in 1000 ml 90%igem Isopropanol 0,5 Stunden bei Rückflusstemperatur gehalten. Nach dem Abkühlen auf Raumtemperatur wurde der Feststoff abgetrennt, mit 700 ml 90%igem Isopropanol gewaschen und im Vakuum bei 70 °C getrocknet. Das so gereinigte Hydrochlorid wurde in 600 ml Methanol bei 50 °C mit 60 ml 30%iger Natronlauge versetzt. Es wurde noch eine Stunde bei 50 °C gerührt und der Feststoff dann nach dem Abkühlen auf Raumtemperatur abgetrennt, mit 200 ml Wasser gewaschen und im Vakuum bei 40 °C getrocknet. Die so erhaltene Base wurde in 250 ml Methanol eine Stunde bei Rückflusstemperatur gehalten. Nach dem Abkühlen auf Raumtemperatur wurde das Festprodukt abgetrennt, mit 75 ml Methanol gewaschen und im Vakuum bei 40 °C getrocknet. Gewonnen wurden 85,3 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C); das sind 20 % der Theorie.

### 3. Vergleichsbeispiel B: Herstellung von Biperiden (Ia)

Zu 2740 ml einer auf 0 °C abgekühlten 1,0-molaren Lösung von Phenylmagnesiumbromid in Diethylether (2,74 mol Phenylmagnesiumbromid) wurden unter Eisbadkühlung 320 g (1,37 mol) des nach 1.2 erhaltenen 3,5:1-Gemischs aus den exo- und endo-Formen des 1-(Bicyclo[2.2.1]hept-5-en-2-yl)-3-piperidino-1-propanons (II) gegeben. Nach beendeter Zugabe wurde das Eisbad entfernt und noch eine Stunde bei Raumtemperatur gerührt. Die Lösung wurde anschließend langsam zu 1500 ml eiskaltem Wasser gegeben und dann drei Mal mit jeweils 500 ml Toluol extrahiert. Die organischen Phasen wurden vereinigt, über Natriumsulfat getrocknet und das Lösungsmittel am Rotationsverdampfer entfernt. Der Eindampfrückstand, 314,3 g (74 % der Theorie) eines Gemischs, das im Wesentlichen aus den Formen Ia bis Id des 1-(Bicyclo[2.2.1]hept-5-en-2-yl)-1-phenyl-3-piperidino-1-propanols (I) im Verhältnis (GC) von 6,1:3,4:1,7:1 bestand, wurde in 3500 ml 90%igem heißen Isopropanol gelöst und die Lösung bei 60 °C mit 228 ml einer 6-molaren Lösung von Chlorwasserstoff in Isopropanol versetzt. Nach der Säurezugabe wurde eine Stunde bei 60 °C und dann noch 0,5 Stunden bei Rückflusstemperatur gerührt. Nach dem Abkühlen auf Raumtemperatur wurden die ausgefallenen Kristalle abgetrennt, mit 700 ml 90%igem Isopropanol gewaschen und im Vakuum bei 70 °C getrocknet. Das so erhaltene Hydrochlorid wurde in 1000 ml 90%igem Isopropanol 0,5 Stunden bei Rückflusstemperatur gehalten. Nach dem Abkühlen auf Raumtemperatur wurde der Feststoff abgetrennt, mit 700 ml 90%igem Isopropanol gewaschen und im Vakuum bei 70 °C getrocknet. Das so gereinigte Hydrochlorid wurde in 600 ml Methanol bei 50 °C mit 60 ml 30%iger Natronlauge versetzt. Es wurde noch eine Stunde bei 50°C gerührt und der Feststoff dann nach dem Abkühlen auf Raumtemperatur abgetrennt, mit 200 ml Wasser gewaschen und im Vakuum bei 40 °C getrocknet. Die so erhaltene Base wurde in 250 ml Methanol eine Stunde zum Rückfluss erhitzt. Nach dem Abkühlen auf Raumtemperatur wurde das Festprodukt abgetrennt, mit 75 ml Methanol gewaschen und im Vakuum bei 40 °C getrocknet. Gewonnen wurden 42,7 g Biperiden (Ia) als farblose Kristalle vom Schmelzpunkt 112 bis 114 °C (Ullmanns Enzyklopädie der techn. Chemie, 4. Aufl., Band 21, Verlag Chemie, 1982, S. 627: 112 - 114 °C) ; das sind 10 % der Theorie.

### 4. Herstellung des Biperidenhydrochlorids

6,7 g Biperiden (Ia) wurden in 75 ml Isopropanol durch Erwärmen auf Rückflusstemperatur gelöst. Die Lösung wurde heiß filtriert und das Filter mit 7 ml Isopropanol nachgewaschen. Die vereinigten Filtrate wurden bei 75 °C mit 4,7 ml 5-molarer Salzsäure versetzt. Anschließend wurde das Gemisch noch 15 Minuten zum Rückfluss erhitzt. Nach dem Abkühlen auf Raumtemperatur wurde die ausgefallene Festsubstanz abgesaugt, mit 7 ml Isopropanol gewaschen und im Vakuum bei 70 °C getrocknet. Erhalten wurden 7,3 g Biperidenhydrochlorid in Form farbloser Kristalle vom Schmelzpunkt 278 bis 280 °C (Ullmanns Enzyklopädie der techn. Chemie, 4. Aufl., Band 21, Verlag Chemie, 1982, S. 627: 278 - 280 °C); das sind 98 % der Theorie.

## Patentansprüche

1. Verfahren zur Herstellung von Biperiden, **dadurch gekennzeichnet, dass** man ein exo/endo-Gemisch von 1-(Bicyclo[2.2.1]hept-5-en-2-yl)-3-piperidino-1-propanon (II) 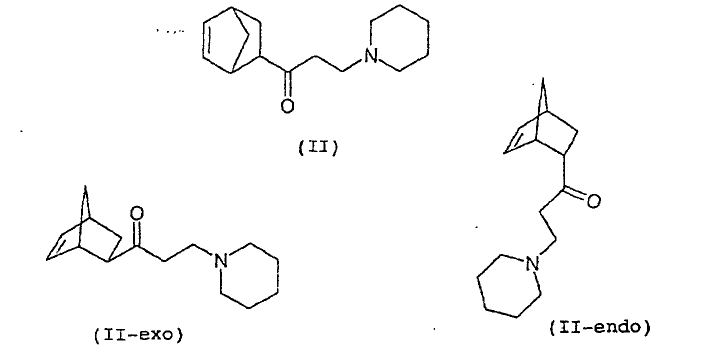 mit einem isomerenverhältnis der exo-Form zur endo-Form von wenigstens 2,5:1 mit Diphenylmagnesium umsetzt, wobei man ein Biperiden-haltiges Isomerengemisch erhält, aus dem man Biperiden (Ia) gewinnt

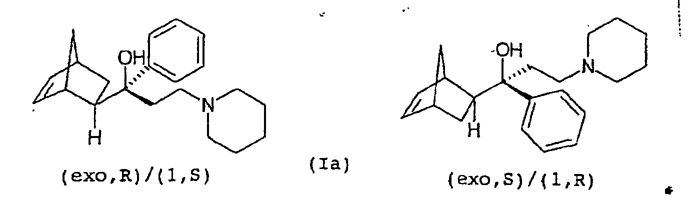

2. Verfahren nach Anspruch 1, **dadurch gekennzeichnet, dass** man Diphenylmagnesium und 1-(Bicyclo[2.2.1]hept-5-en-2-yl)-3-piperidino-1-propanon (II) in einem Molverhältnis von 0,8:1 bis 3:1 einsetzt.

3. Verfahren nach einem der vorhergehenden Ansprüche, **dadurch gekennzeichnet, dass** man die Umsetzung von Diphenylmagnesium mit 1-(Bicyclo[2.2.1]hept-5-en-2-yl)-3-piperidino-1-propanon (II) bei einer Temperatur von -20 °C bis zur Siedetemperatur des Reaktionsgemischs durchführt.

4. Verfahren nach Anspruch 3, **dadurch gekennzeichnet, dass** man die Umsetzung von Diphenylmagnesium mit 1-(Bicyclo[2.2.1]-hept-5-en-2-yl)-3-piperidino-1-propanon (II) bei einer Temperatur von -10 °C bis 90 °C durchführt.

5. Verfahren nach einem der vorhergehenden Ansprüche, **dadurch gekennzeichnet, dass** man die Umsetzung von Diphenylmagnesium mit 1-(Bicyclo[2.2.1]hept-5-en-2-yl)-3-piperidino-1-propanon (II) in Anwesenheit eines cyclischen Ethers durchführt.

6. Verfahren nach einem der vorhergehenden Ansprüche, **dadurch gekennzeichnet, dass** man 1-(Bicyclo[2.2.1]hept-5-en-2-yl)-3-piperidino-1-propanon (II) zu einer Lösung von Diphenylmagnesium hinzufügt.

7. Verfahren nach einem der vorhergehenden Ansprüche, **dadurch gekennzeichnet, dass** man zur Gewinnung von Biperiden (Ia) das bei der Umsetzung des exo/endo-Gemischs von 1-(Bicyclo-[2.2.1]hept-5-en-2-yl)-3-piperidino-1-propanon (II) mit Diphenylmagnesium erhaltene Isomerengemisch von 1-(Bicyclo-[2.2.1]hept-5-en-2-yl)-1-phenyl-3-piperidino-1-propanol (I) 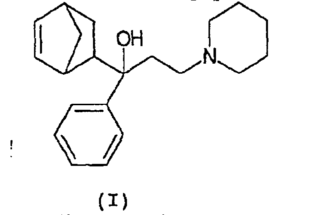
- bei erhöhter Temperatur in wässrigem Isopropanol in das Hydrochlorid überführt,
- das ausgefallene Hydrochlorid von I isoliert, bei erhöhter Temperatur in wässrigem Isopropanol suspendiert und nach dem Abkühlen erneut isoliert,
- das so gewonnene Hydrochlorid von I bei erhöhter Temperatur in einem C₁-C₂-Alkanol oder in einem Gemisch davon mit einer Base in die korrespondierende freie Base I überführt,
- nach dem Abkühlen die gebildete Base I isoliert und mit Wasser wäscht,
- die so erhaltene Base I bei erhöhter Temperatur in einem C₁-C₂-Alkanol oder in einem Gemisch davon suspendiert und nach dem Abkühlen durch Abtrennen des Feststoffs von der Mutterlauge Biperiden (Ia) isoliert.

8. Verfahren nach einem der vorhergehenden Ansprüche, **dadurch gekennzeichnet, dass** man 1-(Bicyclo[2.2.1]hept-5-en-2-yl)-3-piperidino-1-propanon (II) herstellt, indem man
a) Cyclopentadien und Methylvinylketon miteinander umsetzt und ein exo/endo-Gemisch von 1-(Bicyclo[2.2.1]hept-5-en-2-yl)ethanon (III) erhält, 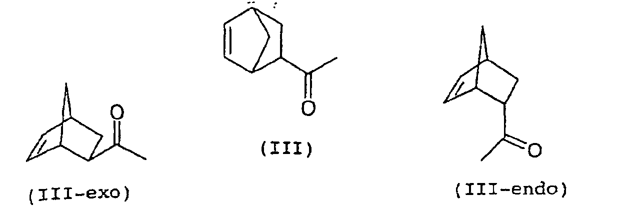
b) dieses Isomerengemisch mit einem Alkali-C₁-C₄-Alkoholat erhitzt und durch fraktionierte Destillation exo-1-(Bicyclo[2.2.1]hept-5-en-2-yl)ethanon (III-exo) gewinnt,
c) dieses exo-1-(Bicyclo[2.2.1]hept-5-en-2-yl)ethanon (III-exo) in Gegenwart einer Säure mit Piperidin und einer Formaldehydquelle umsetzt und ein exo/endo-Gemisch von 1-(Bicyclo[2.2.1]hept-5-en-2-yl)-3-piperidino-1-propanon (II) erhält.

9. Verfahren nach Anspruch 8, **dadurch gekennzeichnet, dass** man in Schritt b) 0,1 bis 5 Gew.-% Alkalialkoholat bezogen auf das Gesamtgewicht der Mischung einsetzt.

10. Verfahren nach Anspruch 8 oder 9, **dadurch gekennzeichnet, dass** man in Schritt b) 10 Minuten bis 5 Stunden bei einem Druck von 1 bis 100 mbar zum Rückfluss erhitzt.

11. Verfahren nach einem der Ansprüche 8 bis 10, **dadurch gekennzeichnet, dass** man in Schritt b) zur Gewinnung von exo-1-(Bicyclo[2.2.1]hept-5-en-2-yl)ethanon (III-exo) über eine Kolonne bei einem Druck von 1 bis 100 mbar und einer Temperatur von 30 bis 110 °C destilliert.

12. Verfahren nach Anspruch 8, **dadurch gekennzeichnet, dass** man in Schritt b) Natriummethanolat verwendet.

13. Verfahren nach einem der Ansprüche 8 bis 12, **dadurch gekennzeichnet, dass** man in Schritt c) Paraformaldehyd als Formaldehydquelle verwendet.

14. Verfahren nach Anspruch 13, **dadurch gekennzeichnet, dass** man in Schritt c) Piperidinhydrochlorid verwendet.

15. Verfahren nach einem der vorhergehenden Ansprüche 8 bis 14, **dadurch gekennzeichnet, dass** man in Schritt c) Formaldehyd im Überschuss einsetzt.

## Claims

1. A method of producing biperides, **characterized in that** an exo/endo mixture of 1-(bicyclo[2.2.1]hept-5-en-2-yl)-3-piperidino-1-propanone (II) 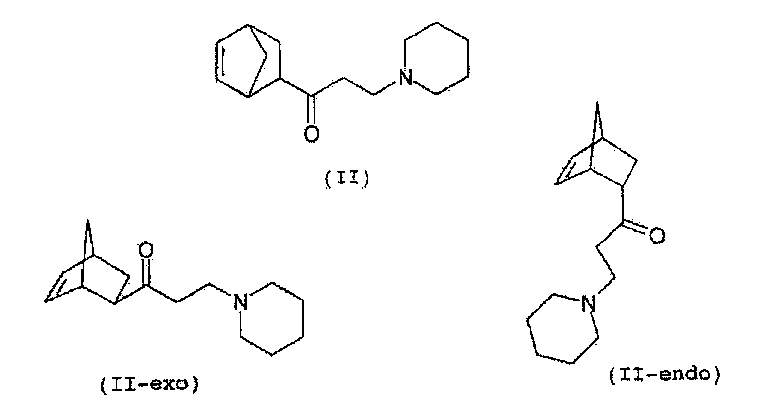 having an isomer ratio of exo form to endo form of at least 2.5:1 is reacted with diphenylmagnesium, a biperide containing isomer mixture being obtained, from which biperides (Ia) are collected

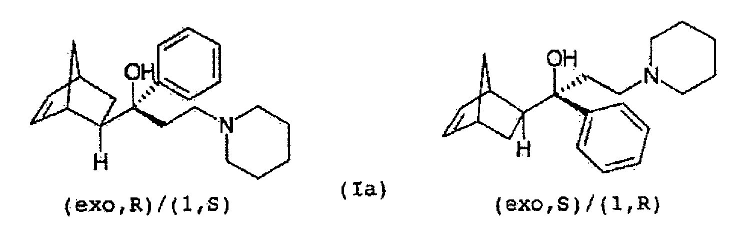

2. The method according to claim 1, **characterized in that** diphenylmagnesium and 1-(bicyclo[2.2.1]hept-5-en-2-yl)-3-piperidino-1-propanone (II) are used in a molar ratio of 0.8:1 to 3:1.

3. The method according to any of the previous claims, **characterized in that** the reaction of diphenylmagnesium with 1-(bicyclo[2.2.1]hept-5-en-2-yl)-3-piperidino-1-propanone (II) is carried out at a temperature of -20°C to the boiling temperature of the reaction mixture.

4. The method according to claim 3, **characterized in that** the reaction of diphenylmagnesium with 1-(bicyclo[2.2.1]-hept-5-en-2-yl)-3-piperidino-1-propanone (II) is carried out at a temperature of -10°C to 90°C.

5. The method according to any of the preceding claims, **characterized in that** the reaction of diphenylmagnesium with 1-(bicyclo[2.2.1]hept-5-en-2-yl)-3-piperidino-1-propanone (II) is carried out in the presence of a cyclic ether.

6. The method according to any of the preceding claims, **characterized in that** 1-(bicyclo[2.2.1]hept-5-en-2-yl)-3-piperidino-1-propanone (II) is added to a solution of diphenylmagnesium.

7. The method according to any of the preceding claims, **characterized in that** for obtaining biperides (Ia) the isomer mixture, obtained when reacting the exo/endo mixture of 1-(bicyclo-[2.2.1]hept-5-en-2-yl)-3-piperidino-1-propanone (II) with diphenylmagnesium, of 1-(bicyclo-[2.2.1]hept-5-en-2-yl)-1-phenyl-3-piperidino-1-propanol (I) 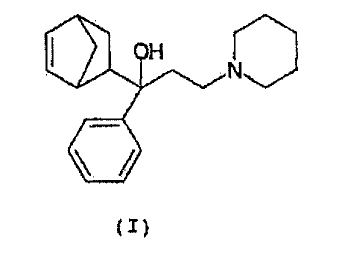
- is converted into the hydrochloride at elevated temperature in aqueous isopropanol,
- the precipitated hydrochloride of I is isolated, suspended in aqueous isopropanol at elevated temperature and isolated again following cooling,
- the resulting hydrochloride of I is converted into the corresponding free base I at elevated temperature in a C₁-C₂ alkanol or in a mixture thereof using a base,
- after cooling down, base I formed is isolated and washed with water,
- the resulting base I is suspended at elevated temperature in a C₁-C₂ alkanol or a mixture thereof and following cooling biperides (Ia) are isolated by separating the solid from the mother liquor.

8. The method according to any of the preceding claims, **characterized in that** 1-(bicyclo[2.2.1]hept-5-en-2-yl)-3-piperidino-1-propanone (II) is produced by
a) reacting cyclopentadiene and methylvinylketone with each other and obtaining an exo/endo mixture of 1-(bicyclo[2.2.1]hept-5-en-2-yl)ethanone (III) 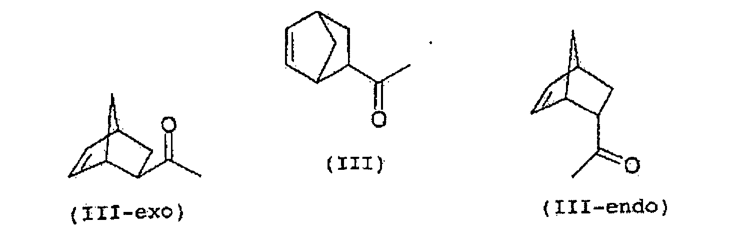
b) heating this isomer mixture with an alkali-C₁-C₄-alcoholate and obtaining exo-1-(bicyclo[2.2.1]hept-5-en-2-yl)ethanone (III-exo) by fractional distillation,
c) reacting this exo-1-(bicyclo[2.2.1]hept-5-en-2-yl)ethanone (III-exo) in the presence of an acid with piperidine and a formaldehyde source and obtaining an exo/endo mixture of 1-(bicyclo[2.2.1]hept-5-en-2-yl)-3-piperidino-1-propanone (II).

9. The method according to claim 8, **characterized by** using in step b) 0.1 to 5 % by weight of alkali alcoholate, based on the total weight of the mixture.

10. The method according to claim 8 or 9, **characterized in that** in step b) heating to reflux is carried out for 10 minutes to 5 hours at a pressure of 1 to 100 mbar.

11. The method according to any of claims 8 to 10, **characterized in that** in step b) distillation is carried out on a column at a pressure of 1 to 100 mbar and a temperature of 30 to 110°C to obtain exo-1-(bicyclo[2.2.1]hept-5-en-2-yl)ethanone (III-exo).

12. The method according to claim 8, **characterized in that** sodium methanolate is used in step b).

13. The method according to any of claims 8 to 12, **characterized in that** in step c) paraformaldehyde is used as formaldehyde source.

14. The method according to claim 13, **characterized in that** piperidine hydrochloride is used in step c).

15. The method according to any of the preceding claims 8 to 14, **characterized in that** in step c) formaldehyde is used in excess.

## Revendications

1. Procédé de préparation du bipéridène, **caractérisé en ce qu'**on fait réagir avec du diphényl-magnésium un mélange des isomères exo et endo, en un rapport exo/endo d'au moins 2,5/1, de la 1-(bicyclo[2.2.1]hept-5-én-2-yl)-3-pipéridino-1-propanone, de formule (II) : 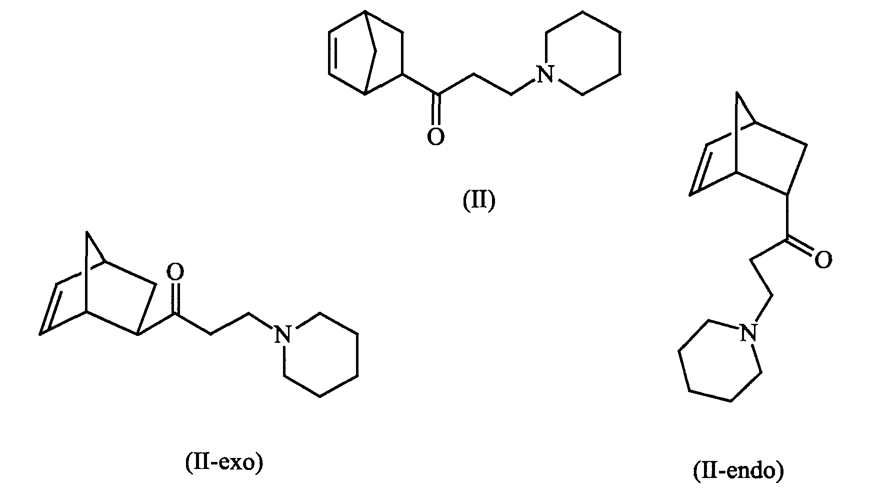 grâce à quoi l'on obtient un mélange d'isomères qui contient du bipéridène et à partir duquel on récupère le bipéridène de formule (Ia) :

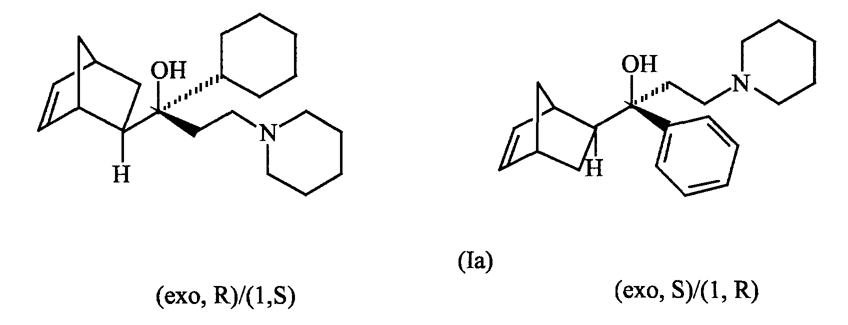

2. Procédé conforme à la revendication 1, **caractérisé en ce qu'**on fait réagir le diphényl-magnésium et la 1-(bicyclo[2.2.1]hept-5-én-2-yl)-3-pipéridino-1-propanone (II) en un rapport molaire de 0,8/1 à 3/1.

3. Procédé conforme à l'une des revendications précédentes, **caractérisé en ce que** l'on effectue la réaction du diphényl-magnésium et de la 1-(bicyclo[2.2.1]hept-5-én-2-yl)-3-pipéridino-1-propanone (II) à une température qui vaut de -20 °C au point d'ébullition du mélange réactionnel.

4. Procédé conforme à la revendication 3, **caractérisé en ce que** l'on effectue la réaction du diphényl-magnésium et de la 1-(bicyclo[2.2.1]hept-5-én-2-yl)-3-pipéridino-1-propanone (II) à une température qui vaut de -10 à 90 °C.

5. Procédé conforme à l'une des revendications précédentes, **caractérisé en ce que** l'on effectue la réaction du diphényl-magnésium et de la 1-(bicyclo[2.2.1]hept-5-én-2-yl)-3-pipéridino-1-propanone (II) en présence d'un éther cyclique.

6. Procédé conforme à l'une des revendications précédentes, **caractérisé en ce que** l'on ajoute la 1-(bicyclo[2.2.1]hept-5-én-2-yl)-3-pipéridino-1-propanone (II) à une solution de diphényl-magnésium.

7. Procédé conforme à l'une des revendications précédentes, **caractérisé en ce que**, pour récupérer le bipéridène (Ia) :
- on convertit en chlorhydrate, à température élevée et dans un mélange d'isopropanol et d'eau, le mélange d'isomères de 1-(bicyclo[2.2.1]hept-5-én-2-yl)-1-phényl-3-pipéridino-1-propanol (I) 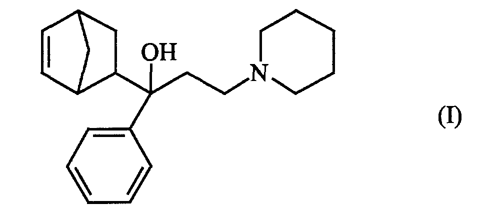 obtenu par réaction du mélange d'isomères exo et endo de la 1-(bicyclo[2.2.1]hept-5-én-2-yl)-3-pipéridino-1-propanone (II) et du diphényl-magnésium,
- on isole le précipité de chlorhydrate de (I), on le remet en suspension dans un mélange d'isopropanol et d'eau, à température élevée, et on l'isole à nouveau après refroidissement de la suspension,
- on convertit le chlorhydrate de (I) ainsi obtenu en la base libre (I) correspondante à l'aide d'une base, à température élevée, dans un alcanol en C₁₋₂ ou dans un mélange de ces alcanols,
- après refroidissement du mélange, on isole la base (I) formée, puis on la lave avec de l'eau,
- on met la base (I) ainsi obtenue en suspension, à température élevée, dans un alcanol en C₁₋₂ ou dans un mélange de ces alcanols, et après refroidissement de la suspension, on isole le bipéridène (Ia) en séparant le produit solide d'avec la solution-mère.

8. Procédé conforme à l'une des revendications précédentes, **caractérisé en ce qu'**on prépare la 1-(bicyclo[2.2.1]hept-5-én-2-yl)-3-pipéridino-1-propanone (II) :
a) en faisant réagir l'un sur l'autre du cyclopentadiène et de la méthyl-vinylcétone, ce qui donne un mélange d'isomères exo et endo de la 1-(bicyclo[2.2.1]hept-5-én-2-yl)-éthanone, de formule (III) : 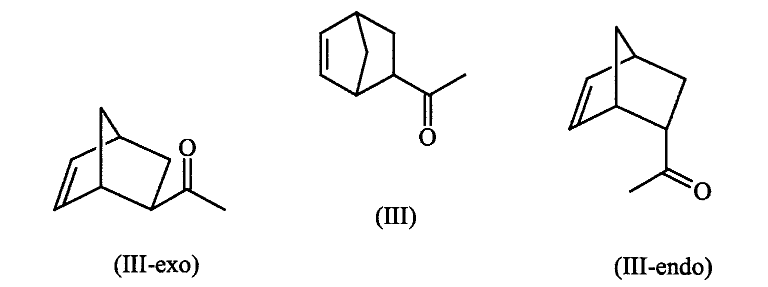
b) en chauffant ce mélange d'isomères avec un alcoolate en C₁₋₄ de métal alcalin et en récupérant, par distillation fractionnée, l'isomère exo de la 1-(bicyclo-[2.2.1]hept-5-én-2-yl)-éthanone (III-exo),
c) et en faisant réagir cet isomère exo-1-(bicyclo-[2.2.1]hept-5-én-2-yl)-éthanone (III-exo) avec de la pipéridine et une source de formaldéhyde, en présence d'un acide, ce qui donne un mélange d'isomères exo et endo de la 1-(bicyclo[2.2.1]hept-5-én-2-yl)-3-pipéridino-1-propanone (II).

9. Procédé conforme à la revendication 8, **caractérisé en ce que**, dans l'étape (b), on emploie de 0,1 à 5 % d'alcoolate de métal alcalin, en poids rapporté au poids total du mélange.

10. Procédé conforme à la revendication 8 ou 9, **caractérisé en ce que**, dans l'étape (b), on chauffe le mélange au reflux sous une pression de 1 à 100 mbar, pendant 10 minutes à 5 heures.

11. Procédé conforme à l'une des revendications 8 à 10, **caractérisé en ce que**, dans l'étape (b), pour récupérer l'exo-1-(bicyclo-[2.2.1]hept-5-én-2-yl)-éthanone (III-exo), on réalise une distillation sur colonne, sous une pression de 1 à 100 mbar et à une température de 30 à 110 °C.

12. Procédé conforme à la revendication 8, **caractérisé en ce que**, dans l'étape (b), on emploie de l'éthanolate de sodium.

13. Procédé conforme à l'une des revendications 8 à 12, **caractérisé en ce que**, dans l'étape (c), la source de formaldéhyde employée est du para-formaldéhyde.

14. Procédé conforme à la revendication 13, **caractérisé en ce que**, dans l'étape (c), on emploie du chlorhydrate de pipéridine.

15. Procédé conforme à l'une des revendications 8 à 14 précédentes, **caractérisé en ce que**, dans l'étape (c), le formaldéhyde est utilisé en excès.
